# EUROPEAN PATENT APPLICATION

(11) **EP 2 708 187 A1**
(43) Date of publication of application: **19.03.2014**
(21) Application number: 13184866.5
(22) Date of filing: 18.09.2013
(51) Int. Cl.: A61B 5/22, A63B 23/20

(54) **Pelvic floor rehabilitation system**

(30) Priority: 18.09.2012 ES 201231447
(71) Applicant: Cikautxo, S. Coop., 48710 Berriatua (Vizcaya) (ES)
(72) Inventor: Martin Estefania, Gonzalo, 48710 Berriatua (Vizcaya) (ES); Amostegui Arakistain, Mikel, 48710 Berriatua (Vizcaya) (ES); Miguel Esparza, Jose, 48710 Berriatua (Vizcaya) (ES); Goikoetxea Larrauri, Josu, 48710 Berriatua (Vizcaya) (ES)
(74) Representative: Intès, Didier Gérard André

(57) **Abstract**

The present invention relates to a pelvic floor rehabilitation system used for strengthening the musculature of the pelvic area by means of performing contractions with a specific force and duration, the system comprising a probe (1) which is inserted into the vaginal or anal canal and a control unit (2) establishing wireless communication with the probe (1), and wherein the control unit (2) is connected through the Internet network with a server (8) to which information obtained by the probe is sent (1), the server (8) in turn being connected with a personal terminal (9) which allows analyzing the data obtained by the probe (1) and prepares a computer application with the specific exercises that the patient must perform, said application being downloaded into the control unit (2) through the server (8).

## Description

### Field of the Art

The present invention relates to devices used for strengthening the muscles of the pelvic region, proposing to that end a wireless system which allows rehabilitating the pelvic floor in a manner which is customized to the specific needs of each patient.

### State of the Art

The pelvic floor or perineum is a group of muscles located in the lower part of the abdomen acting as a support for several organs such as the urethra, the bladder or the rectum.

The weakening of the muscles of the pelvic area generally occurred in elderly women or in pregnant women after childbirth; it is related to a series of problems such as urinary incontinence, sexual dysfunctions, or female genital prolapses.

The lack of muscle tone in the pelvic muscle area can be treated or prevented by means of performing specific exercises known as Kegel exercises. For strengthening pelvic muscles, the use of medical devices having a certain weight which are inserted into the vaginal or anal canal is known, such that once inserted the patient must perform a series of contractions of the muscles of the pelvic area in order to keep the device in place.

To improve the results of muscle toning exercises, intravaginal devices tend to incorporate a pressure sensor which allows knowing the force being exerted by the patient thereon, as well as vibrating means which allow the patient to know the exact moment to start each contraction and the duration thereof, in addition to providing the patient with information about where the device is located and the exact location where he/she must perform the contractions. The intravaginal device is connected by means of wiring or wireless communication with a control unit from which the patient can select the time and duration of the contractions to be performed and view the force that he/she is exerting during the contractions.

However, these devices are based on standardized exercises that cannot be adapted to the specific conditions of each patient and, on the other hand, they do not involve any supervision by a doctor or a physical therapist who verifies that the exercises are being performed correctly.

A pelvic floor rehabilitation system which allows patients to have a customized exercise program and which can be supervised by a healthcare professional for adapting the program to the specific needs of each patient is therefore necessary.

### Object of the Invention

The present invention proposes a rehabilitation system for rehabilitating the musculature of the pelvic area which allows providing therapeutic exercises customized to the specific needs of each patient.

The pelvic floor rehabilitation system object of the invention is used for strengthening the musculature of the pelvic area by means of performing contractions with a specific force and duration. The system comprises a probe suitable for being inserted into the vaginal or anal canal and a control unit establishing wireless communication with the probe. The control unit is connected through the Internet network with a server to which the information obtained by the probe is sent, the server in turn being connected with a personal terminal which allows analyzing the data obtained by the probe and prepares a computer application with the specific exercises that the patient must perform, this computer application being downloaded into the control unit through the server.

It has been envisaged that the control unit is an electronic device such as a smart mobile telephone of the type known as smartphones, an electronic tablet or a PDA, or any other device electronic that can be suitably connected with the probe and the server. Therefore, the patient can use his/her own electronic device to control the probe with which he/she performs the pelvic floor rehabilitation exercises.

Likewise, a healthcare professional such as a doctor specializing in gynecology or a physical therapist, for example, can control the progress of the patient from his/her own personal terminal, establishing the exercises that best suit the patient's needs, which the patient can download in a computer application into his/her electronic device through the Internet network.

### Description of the Drawings

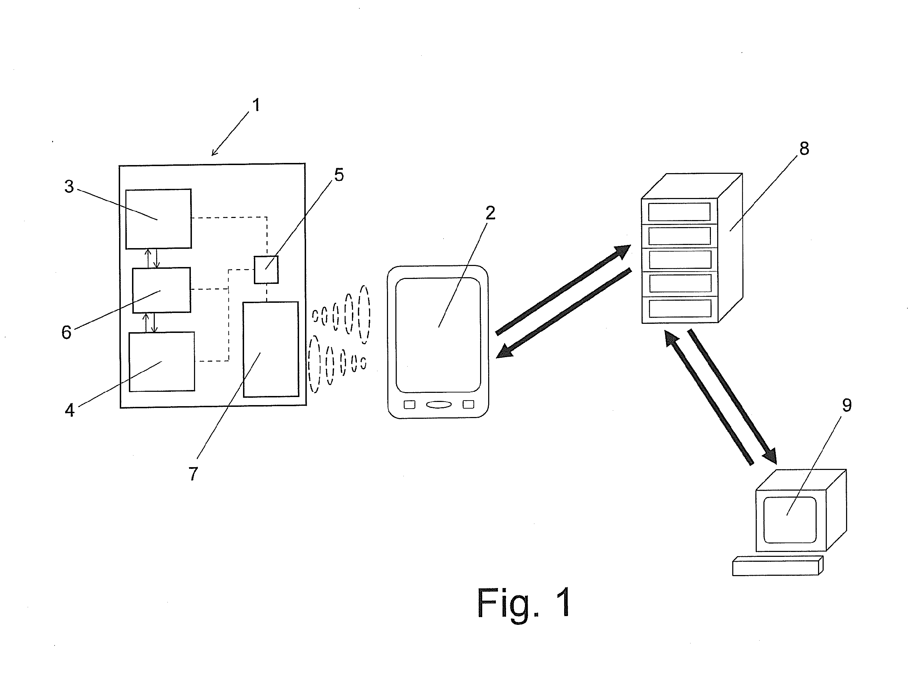

Figure 1 shows a block diagram of the operation of the pelvic floor rehabilitation system proposed by the invention.

### Detailed Description of the Invention

Figure 1 shows a schematic block diagram of the different elements making up the pelvic floor rehabilitation system according to the invention. The system essentially comprises two units between which wireless communication is established. The system thus comprises a probe (1) which is inserted into the vaginal canal or into the anal canal, depending on the patient's gender, and a control unit (2) from which the probe (1) is controlled.

The probe (1) has a pressure sensor (3), a vibrating actuator (4), a battery (5) for powering the different components of the probe (1), a wireless communication device (6) and a processor (7), whereas the control unit (2) is envisaged to be a mobile telephone of the type known as smartphones, although it could also be an electronic tablet, a PDA or any other type of suitable electronic device. In any case, the control unit (2) is a device which can be wirelessly communicated with the probe (1), establishing communication by infrared, WIFI, Bluetooth or Zigbee, or any similar system, from which the probe (1) can be controlled and the variables thereof can be monitored. The possibility that the probe (1) is provided with inflating means has also been envisaged.

Therefore, for exercising the musculature of the pelvic area the patient must perform a series of contractions on the probe (1) with a specific force and duration. The pressure sensor (3) allows knowing the force which is being exerted by the patient on the probe (1) upon contracting the pelvic muscles, whereas the actuator (4) causes the probe (1) to vibrate so that the patient can identify the moment in which he/she must start the contraction and the total time during which he/she must keep the contraction constant. The vibration of the probe (1) also allows stimulating the musculature of the pelvic area and improves the efficiency of the exercises; furthermore the vibration of the probe (1) also allows the patient to identify the exact location of the probe as well as the specific area on which he/she must perform the contractions.

The elements of the control unit (2) are not described in detail since smartphones, electronic tablets or PDAs are electronic devices widely used on the market, said control unit (2) fundamentally having common elements such as a display for viewing data and a touch screen which will allow operating the unit, a wireless communication system (WIFI, infrared, Bluetooth or Zigbee) and/or a 3G mobile telephony system or the like which allows connection to the Internet. Therefore, by means of a specialized computer application, the control unit (2) will allow the patient to perform the exercises that best suit his/her needs, viewing through the display the force with which he/she is performing the contractions as well as the force that must exerted, the total number of contractions to be performed, the time during which he/she must maintain each contraction, the total time of the exercise, etc.

The control unit (2) is communicated through the Internet with a server (8) which is in turn connected with a personal terminal (9) of a healthcare professional such as a gynecologist or a physical therapist, for example. The patient can therefore download from the server (8) the computer application which will be processed in the control unit (2) with a specialized treatment according to his/her specific needs.

The pelvic floor rehabilitation system operates as follows: information obtained in the probe (1) relating to the exercises performed by the patient, such as for example, the number of contractions performed, the time of each contraction, the force exerted in the contractions, the frequency of the contractions, etc., is sent from the control unit (2) to the server (8) That information is stored in the server (8), being accessible by the healthcare professional through his/her personal terminal (9), such that the healthcare professional can perform an individual follow-up on each patient and prepare a specialized program with the exercises to be performed. This specialized program with the specific exercises is sent to the server (8) in a computer application that the patient can download into his/her control unit (2) through the Internet. Therefore, the pelvic floor rehabilitation therapy of the patient is supervised by a healthcare professional at all times, the patient being able to undergo therapy specific to his/her own case.

## Claims

1. A pelvic floor rehabilitation system used for strengthening the musculature of the pelvic area by means of performing contractions with a specific force and duration, comprising a probe (1) which is inserted into the vaginal or anal canal and a control unit (2) establishing wireless communication with the probe (1), **characterized in that** the control unit (2) is connected through the Internet network with a server (8) to which information obtained by the probe is sent (1), the server (8) in turn being connected with a personal terminal (9) which allows analyzing the data obtained by the probe (1) and prepares a computer application with the specific exercises that the patient must perform, said computer application being downloaded into the control unit (2) through the server (8).

2. The pelvic floor rehabilitation system according to claim 1, **characterized in that** the control unit (2) is a smart mobile telephone, an electronic tablet or a PDA.
